# EUROPEAN PATENT APPLICATION

(11) **EP 3 466 398 A1**
(43) Date of publication of application: **10.04.2019**
(21) Application number: 17306339.7
(22) Date of filing: 05.10.2017
(51) Int. Cl.: A61K 8/04, A61K 8/42, A61K 8/34, A61K 8/92, A61Q 19/00, C07C 233/05, B01J 13/00

(54) **N-HYDROCARBYLDIAMIDE COMPOSITION WITH GELATING PROPERTIES**

(71) Applicant: RHODIA OPERATIONS, 75009 Paris (FR)
(72) Inventor: HERVE, Pascal, 33400 Talence (FR)
(74) Representative: Delenne, Marc

(57) **Abstract**

A N-hydrocarbyldiamide composition comprising
a) a compound of formula (Ia) 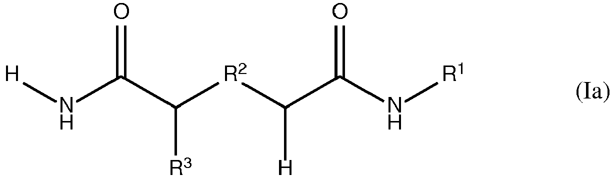 wherein R¹ is selected from linear or branched or cyclic, saturated or unsaturated, hydrocarbon chains having from 1 to 40 carbon atoms, R² is selected from linear or branched, saturated or unsaturated, hydrocarbon aliphatic chains having from 1 to 15 carbon atoms, and R³ is selected from linear or branched alkyl groups having from 1 to 8 carbon atoms, and
b) a compound of formula (Ib) 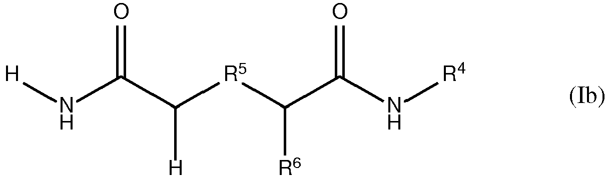 wherein R⁴ is selected from linear or branched or cyclic, saturated or unsaturated, hydrocarbon chain having from 1 to 40 carbon atoms, R⁵ is selected from linear or branched, saturated or unsaturated, hydrocarbon aliphatic chains having from 1 to 15 carbon atoms, and R⁶ is selected from linear or branched alkyl groups having from 1 to 8 carbon atoms
said composition comprising the compounds of formula (Ia) and formula (Ib) in a molar ratio of at least 70:30.

## Description

### TECHNICAL FIELD

The present invention relates to N-hydrocarbyldiamide compositions, their synthesis and their use as structuring agents for oil and/or solvent based compositions. The present invention is also directed to oil and/or solvent based compositions thickened or gelified owing to said N-hydrocarbyldiamide compositions.

### BACKGROUND ART

Numerous fields use non aqueous oil or solvent based formulations in a thickened or gelled form as required by the application from paints, inks, agricultural actives formulations, to make up removers, lubricants and greases, industrial cleaning gels, cables filling compounds. In each of these applications one must adjust formulations to optimize their rheological properties.

To thicken or gelify oils or non-aqueous solvents, it is known to those skilled in the art that organogellants or low molecular weight organic gelators may be used because of their ability to self-assemble into entangled three-dimensional (3D) network structures driven by multiple, weak intermolecular forces such as π-π stacking, van der Waals, electrostatic, metal coordination, charge transfer, and H bonding interactions.

Many such gelling agents (also named gelators) have been developed and described in the literature.

EP 2 254 126 describes organogelator compounds which can be used in combination with an oil in order to form a dielectric fluid used in electric cable. The organo-gelator compounds used in this document may be selected from urea-based compounds, amide-based compounds or mixtures thereof. This document only illustrates the efficiency of urea-based compounds or of aromatic tri-amides as organogelator.

US 2008/0306292 discloses the compound 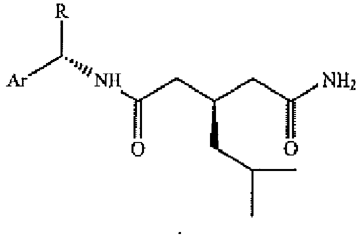 which is used as a starting material for the synthesis of (S)-pregabalin.

The present invention aims at providing new compositions with remarkable gelling properties which are useful in many applications.

Unexpectedly, the inventors have discovered that compositions comprising a mixture of asymmetric N-alkyl diamide compounds in a certain molar ratio are gelling agents of choice.

### SUMMARY OF THE INVENTION

According to a first embodiment, the present invention relates to an N-hydrocarbyldiamide composition comprising
a) a compound of formula (Ia) 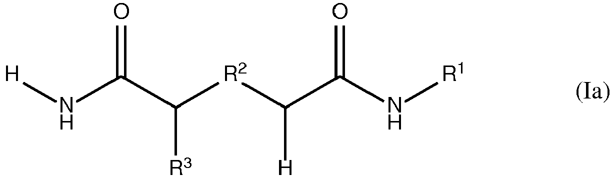 wherein
   R¹ is selected from linear or branched or cyclic, saturated or unsaturated, hydrocarbon chains having from 1 to 40 carbon atoms,
   R² is selected from linear or branched, saturated or unsaturated, hydrocarbon aliphatic chains having from 1 to 15 carbon atoms, and
   R³ is selected from linear or branched alkyl groups having from 1 to 8 carbon atoms, and
b) a compound of formula (Ib) 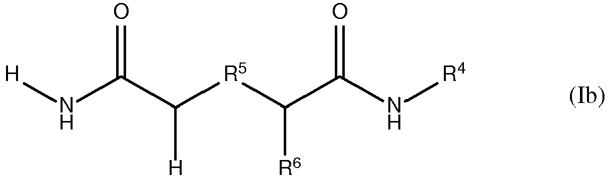 wherein
   R⁴ is selected from linear or branched or cyclic, saturated or unsaturated, hydrocarbon chains having from 1 to 40 carbon atoms,
   R⁵ is selected from linear or branched, saturated or unsaturated, hydrocarbon aliphatic chains having from 1 to 15 carbon atoms, and
   R⁶ is selected from linear or branched alkyl groups having from 1 to 8 carbon atoms
   said N-hydrocarbyldiamide composition comprising the compounds of formula (Ia) and formula (Ib) in a molar ratio of at least 70:30.

Another embodiment of the invention is a process for manufacturing the N-hydrocarbyldiamide compositions according to the invention, said process comprising the reaction between an alkylamine and a reactant selected from an imide, a diacid, a diester, a primary diamide, an ester amide, an acid ester.

Another embodiment of the invention is a mixture comprising the N-hydrocarbyldiamide composition according to the invention and at least one solvent, said N-hydrocarbyldiamide composition being partially or fully solubilized in said solvent [hereinafter, mixture (S)].

A still further embodiment of the invention is a gel composition comprising a carrier and either the N-hydrocarbyldiamide composition according to the invention or the mixture (S) according to the invention, said gel composition being characterized in that the N-hydrocarbyldiamide composition(s) is (are) self-assembled in said carrier in order to provide gjellification thereof.

Finally, further embodiments of the invention relate to the gel composition in accordance with the present invention which is a battery electrolyte or a cosmetic product, such as a personal care product.

### DETAILED DESCRIPTION OF THE INVENTION

The present invention is directed to an N-hydrocarbyldiamide composition comprising
a) a compound of formula (Ia) 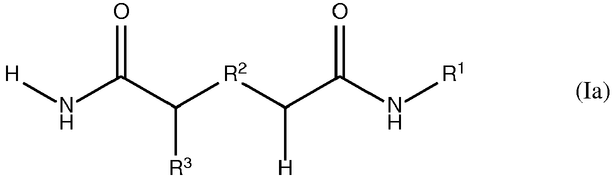 wherein
   R¹ is selected from linear or branched or cyclic, saturated or unsaturated, hydrocarbon chains having from 1 to 40 carbon atoms,
   R² is selected from linear or branched, saturated or unsaturated, hydrocarbon aliphatic chains having from 1 to 15 carbon atoms, and
   R³ is selected from linear or branched alkyl groups having from 1 to 8 carbon atoms, and
b) a compound of formula (Ib) 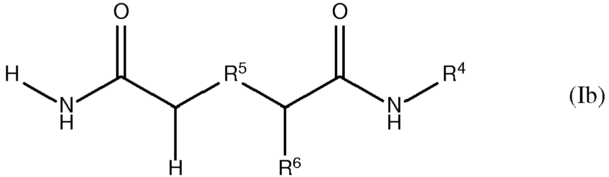 wherein
   R⁴ is selected from linear or branched or cyclic, saturated or unsaturated, hydrocarbon chains having from 1 to 40 carbon atoms,
   R⁵ is selected from linear or branched, saturated or unsaturated, hydrocarbon aliphatic chains having from 1 to 15 carbon atoms, and
   R⁶ is selected from linear or branched alkyl groups having from 1 to 8 carbon atoms
   said N-hydrocarbyldiamide composition comprising the compounds of formula (Ia) and formula (Ib) in a molar ratio of at least 70:30.

In accordance with a preferred embodiment, the molar ratio of the compound of formula (Ia) to compound (Ib) is in the range of from 70:30 to 99:1, preferably in the range of from 75:25 to 98:2, more preferably of from 85:15 to 97:3 and most preferably from 90:10 to 97:3.

In the N-hydrocarbyldiamide composition according to the invention the aggregated weight proportion of compounds (Ia) and (Ib) is at least 50wt%, preferably at least 80 wt%, more preferably at least 90 wt% of the total weight of the composition. Even more preferably the composition essentially consists of compounds (Ia) and (Ib) or consists of compounds (Ia) and (Ib).

Preferred in accordance with the invention are compositions in which R¹ is identical to R⁴, R² is identical to R⁵ and R³ is identical to R⁶, i.e. the N-hydrocarbyldiamide composition is preferably an isomer mixture.

Within the meaning of the present invention, by "hydrocarbon chain", it is to be understood a hydrocarbon chain comprising carbon atoms and hydrogen atoms, wherein said hydrocarbon chain may optionally be substituted by or contain one or more heteroatoms, such as oxygen atoms.

Within the meaning of the present invention, "aliphatic chain" is to be understood as a non-aromatic chain.

According to an embodiment of the invention, R¹ or R⁴ of the compound of formula (Ia) respectively formula (Ib) is a hydrocarbon chain constituted only of carbon atoms and hydrogen atoms.

According to another embodiment of the invention, R¹ or R⁴ of the compound of formula (Ia) respectively formula (Ib) is a linear, branched or cyclic aliphatic (non-aromatic) hydrocarbon chain.

According to an embodiment of the invention, R¹ or R⁴ is selected from a linear or branched, saturated or unsaturated, hydrocarbon chain having from 2 to 40 carbon atoms, preferably from 4 to 32 carbon atoms, more preferably from 5 to 24 carbon atoms, even more preferably from 6 to 18 carbon atoms.

Just by way of example, R¹ and/or R⁴ can preferably be selected from pentyl, hexyl, octyl, decyl, dodecyl, tetradecyl, palmityl, stearyl, 12-hydroxy stearyl, oleyl, 12-hydroxyloleyl, linoleyl, linolenyl, arachidyl or behenyl groups.

R² and/or R⁵ are preferably selected from hydrocarbon aliphatic chains comprising a hydrocarbon main chain having from 1 to 14 carbon atoms. R² and/or R⁵ preferably comprise from 1 to 12 carbon atoms, more preferably from 1 to 4 carbon atoms and still more preferably from 1 to 2 carbon atoms.

Besides, R² and/or R⁵ are preferably selected from (i) linear alkylene chains and (ii) from linear alkylene chains substitued by one and only one linear alkyl group, wherein the linear alkyl group substituent comprises a number of carbon atoms that is preferably lower, more preferably does not comprise more than half of the carbon atoms of the linear alkylene chains and more preferably does not exceed two and more preferably does not exceed one carbon atom. More preferably, R² and/or R⁵ are linear alkylene chains.

Most preferably, R² and/or R⁵ are methylene.

Other possible choices for R² and/or R⁵ include
-(CH₂)ᵢ-, wherein I is 2, 3, 4, 5, 6, 7, 8, 9 or 10, preferably 1 or 2
-CH(CH₃)-(CH₂)ⱼ- wherein j is 1, 2, 3, 4, 5, 6, 7, 8 or 9, preferably 1 or 2 and
-(CH₂)ₖ-C(CH₃)-(CH₂)ₖ'- wherein k and k' are integers equal to or exceeding 1 and the sum of k and k' is 2, 3, 4, 5, 6, 7, 8, or 9.

R² and R⁵ may also be -CH(CH₂-CH₃)-CH₂.

R³ and R⁶ are linear or branched alkyl groups having from 1 to 8, preferably from 1 to 4 carbon atoms, most preferably R³ and R⁶ are methyl.

Preferably, the compounds of formula (Ia) and (Ib) forming the N-hydrocarbyldiamide composition according to the invention do not present urea functions of type -NH-CO-NH-.

The present invention is also directed to a process for manufacturing the N-hydrocarbyldiamide compositions in accordance with the invention, said process comprising the reaction between at least one amine and at least one reactant selected from an imide, a diacid, a diester, a primary diamide, an ester amide, an acid ester or other combinations.

The process for manufacturing the N-hydrocarbyldiamide composition according to the invention comprises the steps of
a) the reaction between an amine and a reactant selected from an imide, a diacid, a diester, a primary diamide, an ester amide or an acid ester, and thereafter
b) subjecting the reaction product obtained in step a) to at least two cycles of recrystallization and washing steps

Ammonia may also be added during the process if it is not already present in the molecule as amide or imide functions. Ammonia may allow favoring the manufacture of the mono-alkyl form (versus the dialkyl form) of the diamide and may also allow favoring the elimination of by-products formed during the reaction (such as alcohol coming from reaction with ester as a co-product).

General processes for manufacturing diamides are described in WO 2010/031867.

In accordance with a preferred embodiment, the process in accordance with the invention comprises the reaction between at least one amine of formula (II) 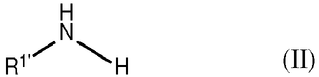 and at least one reactant selected from
a cyclic imide of formula (III) 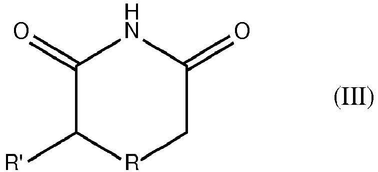 a diacid of formula (IV) 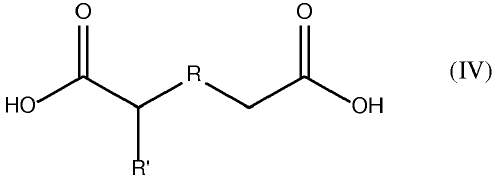
- a diester of formula (V) 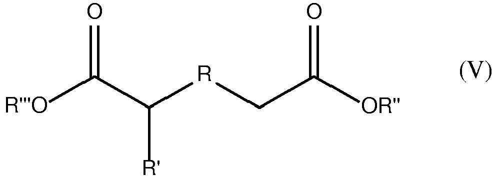
- a primary diamide of formula (VI) 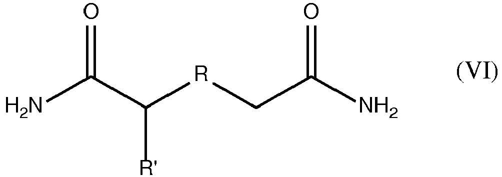
- an ester amide of formula (VII) 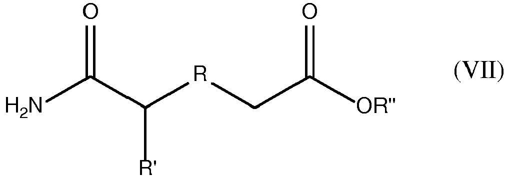 an acid ester of formula (VIII) 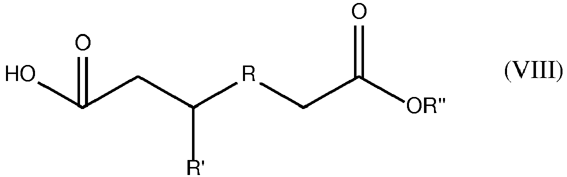 wherein
   R is either R² or R⁵, R² and R⁵ having the same meaning as above in formula (Ia) respectively (Ib),
   R' is either R³ or R⁶, R³ and R⁶ having the same meaning as above in formula (Ia) respectively (Ib),
   R¹' is hydrogen, R¹ or R⁴, R¹ and R⁴ having the same meaning as above in formula (Ia) respectively (Ib),
   and R" and R'", which may be the same or different, represent a hydrocarbon aliphatic chain, saturated or unsaturated, linear or branched, having from 1 to 40 carbon atoms.

In particular, the process of the invention comprises the direct reaction between a cyclic imide of formula (III) and an amine of formula (II).

The compounds of formula (Ia) and (Ib) present in the N-hydrocarbyldiamide compositions according to the invention can thus be obtained by an imide ring-opening reaction of a cyclic imide with addition of the amine.

The imide in this embodiment of the process according to the invention may be preferably selected from 2-methyl-glutarimide, 3-methyl-glutarimide, 2-ethyl-glutarimide, 3-ethyl-glutarimide, 2-methyl-succinimide and 2-ethyl-succinimide.

For example, in order to obtain a compound of formula (Ia) or (Ib) wherein R² respectively R⁵ is -CH₂-, then the imide can be a cyclic imide of formula (III-1):

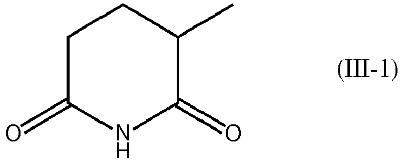

The amine II is a primary amine of general formula R¹'-NH₂, with R¹' defined as above.

The reaction for preparing the compounds of formula (I) according to the invention may be performed for example, at a temperature ranging from 20°C to 200°C, preferably from 50°C to 180°C, more preferably from 100°C to 160°C. In the case of lighter amines, such as amines having less than 6 carbon atoms, the reaction may be performed with a progressive heating in order to avoid loss of amines through evaporation or may be performed under pressure to keep the amine in the liquid phase. For example, at the beginning of the reaction, the temperature of the mixture (ingredients) is approximately equal to the boiling point of the amine.

The reaction for preparing the compounds of formula (I) according to the invention is preferably performed under atmospheric pressure.

In the process according to the invention, the reaction product in step a) is subjected to recrystallization and washing cycles. With increasing number of recrystallization cycles, the ratio of compounds of formula (Ia) and (Ib) increases, if (Ia) and (Ib) are isomers of the same N-hydrocarbyldiamide.

The recrystallization and washing cycles are repeated in a number as neded to achieve the desired molar ratio of compounds of formula (Ia) to formula (Ib).

The present invention is also directed to the use of the N-hydrocarbyldiamide composition according to the invention as a gelling agent (gelator).

It has been surprisingly found that the N-hydrocarbyldiamide composition according to the invention has remarkable gelling properties in very different carriers. The N-hydrocarbyldiamide composition according to the invention allows gelling different kinds of compositions, including non-polar solvents (such as mineral oils, vegetable oils, animal oils and synthetic oils) and polar solvents (such as glycerol and carbonates).

### Mixture (S) containing N-hydrocarbyldiamide compositions

The present invention is also directed to a mixture (S) comprising the N-hydrocarbyldiamide composition according to the invention and at least one solvent, said compound of formula (I) being partially or fully solubilized in said solvent.

In the mixture (S) of the invention, the N-hydrocarbyldiamide composition acording to the invention is preferably fully solubilized in the at least one solvent. The mixture (S) is preferably a solution comprising at least one solvent and, fully dissolved therein, the compounds of formula (Ia) and (Ib), that is to say the mixture (S) is free of any compound that would be insolubilized in the solvent.

Preferably, the mixture (S) is one which, when at room temperature and at atmospheric pressure (20 °C, 1 atm), comprises the N-hydrocarbyldiamide composition according to the invention in partially or fully solubilized form in the solvent. More preferably, the mixture (S) is one which, when at room temperature and at atmospheric pressure, comprises the N-hydrocarbyldiamide composition acording to the invention in fully solubilized form.

Advantageously, the mixture (S) is one which, when at 60°C and at atmospheric pressure, comprises the N-hydrocarbyldiamide composition acording to the invention in fully solubilized form in the solvent. Preferably, the mixture (S) is one which, when at 40°C and at atmospheric pressure, comprises the N-hydrocarbyldiamide composition acording to the invention in fully solubilized form.

Advantageously, the mixture (S) is a solution when it is put at 60°C and at atmospheric pressure. Preferably, the mixture (S) is a solution when it is put at 40°C and at atmospheric pressure. More preferably, the mixture (S) is a solution when it is put at room temperature (20°C) and at atmospheric pressure.

With the meaning of present invention, the solubilized form is different from the self-assembled form.

According to an embodiment, the solvent used in the mixture (S) according to the invention is a good solvent and can be a polar solvent or a mixture of a polar solvent and an apolar solvent. Within the meaning of the present invention, by "polar solvents", it is to be understood solvents having a polar and hydrogen bonding components respectively δₚ and δₕ (Hansen solubility parameter) strictly greater than 0. Hansen solubility parameters are well known for the skilled person: δₚ corresponds to the energy from dipolar intermolecular forces between molecules and δₕ corresponds to the energy from hydrogen bonds between molecules.

According to an embodiment of the invention, the solvent of the mixture (S) is selected from alcohols having from 1 to 10 carbon atoms, preferably selected from methanol, ethanol, isopropanol, butanol, isobutanol, diethylene glycol, butylene glycol, methyl propane diol.

According to an embodiment of the invention, the solvent of the mixture (S) is selected from acetal derivatives having from 2 to 12 carbon atoms, and is preferably selected from the Augeo® family of solvents such as Augeo® SL191 which is a racemic mixture (+/-)-2,2-dimethyl-4-hydroxymethyl-1,3-dioxolane also known as isopropylidene glycerol.

According to an embodiment of the invention, the solvent of the mixture (S) is itself a mixture of (i) alcohols having from 1 to 10 carbon atoms (preferably selected from methanol, ethanol, isopropanol, butanol, isobutanol, diethylene glycol, butylene glycol, methyl propane diol), (ii) alkyl esters having from 2 to 12 carbon atoms (preferably selected from ethyl acetate, propyl acetate, butyl acetate, hexyl acetate, ethylhexyl acetate and octyl acetate) and (iii) acetal derivatives having from 2 to 12 carbon atoms (preferably selected from the Augeo® family of solvents (available from Solvay SA) such as Augeo® SL191 which is a racemic mixture (+/-)-2,2-dimethyl-4-hydroxymethyl-1,3-dioxolane also known as isopropylidene glycerol).

These solvents are capable of dissolving the N-hydrocarbyldiamide compositions of the invention, the latter being generally in a powder form as a solid.

The mixture (S) according to the invention may be from a liquid state to a flowable thick paste at temperatures ranging from 20°C to 50°C.

According to an embodiment of the invention, the N-hydrocarbyldiamide composition of the invention represents from 1% to 75% by weight, preferably from 10% to 50% by weight, more preferably from 15% to 30% by weight, of the total weight of the mixture (S).

According to a particular embodiment, the mixture (S) of the invention comprises an N-hydrocarbyldiamide composition according to the invention, where in compounds (Ia) and (Ib), R¹ and R⁴ differ in chain length. When the mixture (S) of the invention comprises such combination, the use of the mixture (S) is more flexible and can be used in very different carriers in order to allow the gelification of said carriers.

The mixture (S) according to the invention may be obtained by simple mixing of the ingredients (N-hydrocarbyldiamide composition and solvent), preferably at a temperature comprised between 10°C and 60°C, ideally about 25°C (room temperature).

The mixture (S) allows obtaining a final gelled composition more easily, in particular, the final gel composition, when starting from the mixture (S) of the invention, may be obtained by mixing the ingredients at ambient temperature.

The most important advantage of the mixture (S) of the invention is that it allows the gelification of a composition at room temperature (about 25°C), which can be very useful or even necessary if the solvents or oils to be gelified or thickened are too volatile at the high temperature required for the mixing or if there are additives in the composition that are sensitive to high temperatures.

The mixture (S) according to the invention may be used in a composition in order to gelify such compositions.

### Gel composition comprising the N-hydrocarbyldiamide compositions

The present invention is also directed to a gel composition comprising the N-hydrocarbyldiamide composition according to the present invention and at least one carrier, said N-hydrocarbyldiamide composition according to the present invention being in a self-assembled form in said carrier in order to provide gelification thereof.

Within the meaning of the present invention, by "self-assembled form" it is to be understood that the gelator self assembles into entangled three-dimensional (3D) network structures driven by multiple, weak intermolecular forces such as π-π stacking, van der Waals, electrostatic, metal coordination, charge transfer, and H bonding interactions.

According to an embodiment of the invention, the elastic modulus of the gel composition ranges from 0.5 Pa to 500000 Pa. The elastic modulus may be measured with a ARG2® Rheometer from TA instruments, as described in the experimental part.

According to a preferred embodiment of the invention, the N-hydrocarbyldiamide composition according to the present invention represents from 0.01 % to 10% by weight, preferably from 0.05% to 5% by weight, more preferably from 0.1 % to 2.5% by weight, even more preferably from 0.1% to 1 % by weight, of the total weight of the gel composition.

According to a preferred embodiment, the carrier represents from 50% to 99.99% by weight, preferably from 60% to 99.95% by weight, more preferably from 70% to 99.9% by weight, even more preferably from 80% to 99.9% by weight, of the total weight of the gel composition.

According to an embodiment, the carrier is selected from non-polar to polar solvents, preferably selected from mineral oils, vegetable oils, animal oils or synthetic oils. For example, the carrier may be selected from vegetable oils, such as rapeseed oil sometime known as Canola oil, methylated seed oils, such as methylated soy bean oil, linear or iso-alkanes, such as linear or iso- C₄-C₁₈ alkanes, diesters having from 8 to 40 carbon atoms, glycerol, ethyl carbonate and dimethyl carbonate.

The carrier may be selected according to the intended final use of the gelled composition. The gelled composition may be used in cosmetic formulations or in the agricultural coatings or cleaning.

According to an embodiment of the invention, the carrier is chosen among those acceptable in cosmetic formulations.

According to a particular embodiment, the gel composition of the invention comprises at least two or more N-hydrocarbyldiamide compositions according to the invention.

According to an embodiment, the gel composition of the invention further comprises at least one additional additive, preferably selected from surfactants.

There is no specific limitation as to the nature of the surfactant. The skilled person will select suitable surfactants based on his professional knowledge and expertise and taking into account the needs of the specific application case.

Suitable surfactants are commercially available in great variety from a number of suppliers, including Solvay SA, BASF and DOW Chemicals.

Just by way of example, ionic or nonionic surfactants comprising at least one alkylene oxide moiety, preferably surfactants of this type which are free of aromatic rings may be mentioned here.

Non-ionic surfactants selected from the group consisting of polyethylene glycol esters of fatty acids constitute a particularly preferred group of surfactants.

Suitable polyethylene glycol esters of fatty acids surfactants include polyethylene glycol fatty acid monoesters and polyethylene glycol fatty acid diesters, more typically mono- and di-esters of polyethylene glycols and saturated or unsaturated (C₈-C₂₂), more typically (C₁₂-C₁₈), fatty acids and mixtures thereof, such as for example, poly(ethylene glycol) monomyristates, poly(ethylene glycol) monostearates, poly(ethylene glycol) distearates, poly(ethylene glycol) monooleates, poly(ethylene glycol) dioleates, poly(ethylene glycol) linolenates, poly(ethylene glycol) dibehenates, poly(ethylene glycol) monobehenates, poly(ethylene glycol) monoerucates, and their mixtures.

Examples of suitable polyethylene glycol esters of fatty acids are commercialized by Solvay under the names Alkamuls® AP, Alkamuls® A, Alkamuls® VO/2003 and Alkamuls® VO2005.

Another embodiment of the present invention relates to formulations comprising the gel composition with surfactans as described above and a biologically active ingredient.

The term "biologically active ingredient", when used herein, is intended to denote an entity which interacts with biological material in a manner that dieseases in mammals may be prevented or combatted or which influences growth of agricultural crops or which combats weeds, insects, bacteria, fungi in agricultural crops.

There is no principal limitation as to the nature of the biologically active ingredient in the formulation of the present invention.

For the purpose of the invention the biologically active compounds are preferably biologically active compounds used to control agricultural pests and include, for example, herbicides, plant growth regulators, crop dessicants, fungicides, bactericides, bacteriostats, insecticides, and insect repellants.

Preferred agriculturally active materials for use in the formulations according to the invention are abamectin, azamethiphos, azoxystrobin, cyproconazole, bordeaux mixture, carbendazim, chlorsulfuron, copper hydroxide, copper oxide, copper oxychloride, cymoxanil, diflubenzuron, PMP, ethofumesate, DMP, lenacil, fenoxaprop-p-ethyl, iodosulfuron, florasulam, flutriafol, imidacloprid, imidacloprid, b-cyfluthrin, indoxacarb, iprodione, isoproturon, mancozeb, copper oxy, metamitron, nicosulfuron, rimsulfuron, thiacloprid, deltamethrin, thiobendazole, uniconazole, difenconazole, oxyfluorfen, quizalofop-p-ethyl, tebuconazole and their mixtures.

All these additives are selected according to the intended final use of the gelled composition.

According to this embodiment, the additional additive(s) may represent(s) from 0.1% to 45% by weight, preferably from 1% to 40% by weight, more preferably from 5% to 30% by weight, of the total weight of the gel composition.

According to an embodiment of the invention, the gel composition comprises, in particular consists in:
- from 0.01% to 10% by weight, preferably from 0.05% to 5% by weight, more preferably from 0.1% to 2.5% by weight, even more preferably from 0.1% to 1% by weight, of the N-hydrocarbyldiamide composition according to the invention,
- from 50% to 99.99% by weight, preferably from 60% to 99.95% by weight, more preferably from 70% to 99.9% by weight, even more preferably from 80% to 99.9% by weight, of carrier(s),
- up to 45% by weight, preferably from 0.1% to 45% by weight, more preferably from 1% to 40% by weight, even more preferably from 5% to 30% by weight, of additional additive(s).

The gel composition may be prepared either directly from N-hydrocarbyldiamide compositions according to the invention alone or from the mixture (S) according to the invention comprising the N-hydrocarbyldiamide composition according to the invention.

The gel composition may be obtained by adding the compound of formula (I) (gelator) into the carrier for example at a temperature ranging from 50°C to 150°C, preferably from 70°C to 130°C, more preferably from 90°C to 110°C. Preferably, the carrier is agitated. The agitation can be performed using means well known for the skilled person, such as a tip sonicator or a propeller agitation. The agitation may last from 1 to 10 minutes.

If the gel composiions are prepared starting form a mixture S as defined above, the temperature of mixing and compounding the ingredients is preferably 30°C or less.

The gel composition according to the invention may be used in different fields of application, such as in batteries or in personal care products.

Indeed, the gel composition according to the invention may be used as a gel electrolyte in batteries.

The gel composition according to the invention may also be used as a personal care product, in particular for cosmetic applications. Indeed, it has been found that the gelators according to the invention do not lose their gelling properties even in the presence of other functional additives such as surfactants generally found in personal care products.

The N-hydrocarbyldiamide compounds of the present invention have the following advantages:
- they can be used at very low concentrations,
- they allow the formation of strong gel compositions,
- they are multi-purposes in the sense that they allow obtaining a gelled composition based on very different kinds of carriers or solvents from non-polar to polar solvents : the use of different chain length of the gelator allows to adapt said gelator to different solvents or oils. Furthermore, the mixing of gelators with different chain lengths offers another kind of flexibility ;

The use of the mixture (S) of N-alkyl diamide gelators, wherein the gelators are partially or fully in a solubilized form, allow for the gelation at room temperature with simple mixing.

In particular, the gelled compositions obtained owing to the N-hydrocarbyldiamide compounds of the invention are very stable, they keep their gel form for a long time, up to relatively high temperatures. The gelled compositions according to the invention show no syneresis phenomenon for most solvents.

The gelled compositions according to the invention show large elastic and storage moduli even at very low gelator concentrations.

### Examples

### Example 1a (Comparative) : Reparation of a 57/43 mixtures of N-hexyl-4-methylpentanediamide (C4-isomer) and N-hexyl-2-methylpentanediamide (C2 isomer), also named hereafter C6 gelator 57/43)

### Isomer4/Isomer2 Ratio 57/43

In a round bottom flask equipped with a magnetic stirrer and a condenser, 50.0 g of a mixture composed of 2-methylglutarimide (MGI) (86.4 wt%, 0.34 mol) and 2-ethylsuccinimide (10.7 wt%, 0.04 mol), 40.1 g of hexylamine (0.40 mol) and 0.9 g of tBuONa were added.

The mixture was then allowed to stir at 130°C during 2 hours. Over the course of the reaction the mixture became monophasic (yellow solution). The reaction progress is followed thanks to GC analysis and at the end of the reaction, the product was recovered thanks to recrystallization in 150 mL of methyl ethyl ketone (MEK) followed by washing two times with 100 mL of ethyl acetate.

After drying under vacuum, 71.5 g (92% yield based on MGI) of the product was obtained as a white powder (melting point (mp): 111.8 - 115.4 °C).

NMR analysis showed that the product was obtained as a mixture of 2 isomers:
- N¹-hexyl-4-methylpentanediamide (57 wt%),
- N¹-hexyl-2-methylpentanediamide (43 wt%).

### Example 1b (Comparative) Reparation of a 65/35 mixture of N-hexyl-4-methylpentanediamide (C4-isomer) and N-hexyl-2-methylpentanediamide (C2 isomer), also named hereafter C6 gelator 65/35)

In a 500 ml glass reactor equipped with a cooling system, 100.0 g of methyl glutarimide (MGI) and 76.6 g of hexylamine then 1.0 g of tBuONa was added. The reactor was then heated to 130°C and maintained at the reaction temperature for 2.5h. Then the reaction mixture was cooled down. The solid was added into 400 ml of methylethylketone (MEK) and heated to 100°C. The solution was then cooled down and the obtained solid was collected by filtration and washed with ethyl acetate (EA, 2*300ml). The obtained solid was dried in vacuo to give 137.0 g of white solid, the yield was 76%, the purity was >98%.

NMR analysis showed that the product was obtained as a mixture of 2 isomers:
- N1-hexyl-4-methylpentanediamide (65 wt%),
- N1-hexyl-2-methylpentanediamide (35 wt%).

### Example 1c (according to the invention): Reparation of a 97/3 mixture of N-hexyl-4-methylpentanediamide (C4-isomer) and N-hexyl-2-methylpentanediamide (C2 isomer), also named hereafter C6 gelator 97/3)

In a 500 ml round bottom flask equipped with a mechanical stirrer and a condenser 63,57 g of methylglutarimide (0,5 mol) and 50,60 g of n-hexylamine (0,5 mol, 1 eq.) was added. The suspension was heated to 130°C during 4 hours. The mixture was then cooled down to 80°C, then 320 mL of methylethylketone are added. The mixture was heated to 80°C, then cooled down to room temperature, leading to a crystallization. The mixture was filtered and the solid is washed twice with 100 mL of ethyl acetate. The obtained solid contained an isomer 4/isomer 2 ratio of de 54/46. The solid is suspended in 1 l of methylethylketone, the mixture was heated to 80°C, then cooled to 30°C and filtered. The obtained solid contained an isomer 4/ isomer 2 ratio of 75/25. This solid was suspended in 500 ml of methylethylketone heated to 80°C then cooled down to 30°C and filtered. The obtained solid (13,3 g, 12% yield) contained an isomer 4/ isomer 2 ratio of 97/3.

### Example 2a (Comparative): Reparation of a 30/70 mixture of 4-methyl (C4-isomer) and 2-methyl substitued lauryldiamide (C2 isomer), also named hereafter C12 gelator 30/70

In a 1000 ml round bottom flask equipped with a mechanical stirrer and a condenser 127.13 g of methylglutarimide (0.7 mol) and 185.35 g of melted dodecylamine (0.7 mol, 1 eq.) was added. The suspension was heated to 130°C during 4 hours. The obtained product mixture contained an isomer4/isomer2 ratio of 52/48. This mixture was then cooled down to 100°C, then 400 mL of methylethylketone were added. The mixture was then heated to 80°C, then cooled down to room temperature, leading to a crystallization. The mixture was filtered and the solid was washed twice with 100 mL of ethyl acetate. The solid was suspended in 1.5 l of methylethylketone, the mixture was heated to 80°C, then cooled to 30°C and filtered. The **filtrate** was heated to 80°C, then cooled down to room temperature, leading to a crystallization, then filtered. The solid contained an isomer 4/isomer 2 ratio of 30/70.

### Example 2b (Comparative) Reparation of a 55/45 mixture of 4-methyl (C4-isomer) and 2-methyl substitued lauryldiamide (C2 isomer), also named hereafter C12 gelator 55/45

In a 500 ml glass reactor equipped with a cooling system, 68.6 g (0.54 Mol 1eq) of methyl glutarimide (MGI) and 100.0 g (0.54 mol 1eq) of laurylamine then 1.0 g of tBuONa was added. The reactor was then heated to 150°C and maintained at the reaction temperature for 3h. Then the reaction mixture was cooled down.

The solid was added into 400 ml of methylethylketone (MEK) and heated to 100°C. The solution was then cooled down and the obtained solid was collected by filtration and washed by ethyl acetate (2*300ml). The Obtained solid was dried in vacuo to give 135.0 g of white solid, the yield was 80%, the purity was >98%.

NMR analysis showed that the product was obtained as a mixture of 2 isomers:
- N1-hexyl-4-methylpentanediamide (55 wt%),
- N1-hexyl-2-methylpentanediamide (45 wt%).

### Example 2c (Comparative): Reparation of a 58/42 mixture of 4-methyl (C4-isomer) and 2-methyl substitued lauryldiamide (C2 isomer), also named hereafter C12 gelator 58/42

In a round bottom flask equipped with a magnetic stirrer and a condenser, were added 17.0 g of a mixture composed of 2-methylglutarimide (86.4 wt%, 0.12 mol) and 2-ethylsuccinimide (10.7 wt%, 0.01 mol), 25.0 g of dodecylamine (0.13 mol) and 0.4 g of tBuONa.

The mixture was then allowed to stir at 150°C during 2 hours. Over the course of 15 the reaction the mixture turned yellow and reaction progress was followed by GC analysis. After reaction completion, the desired product was recovered by recrystallization in 200 mL of methyl ethyl ketone (MEK) followed by washing two times with 100 mL of ethyl acetate.

After drying under vacuum, 29.1 g (78 % yield based on MGI) of the product was obtained as a white powder (mp: 112.9 - 117.8 °C).

NMR analysis showed that the product was obtained as a mixture of 2 isomers:
- N1-lauryl-4-methylpentanediamide (58 wt%),
- N1-lauryl-2-methylpentanediamide (42 wt%).

### Example 2d (according to the invention): Reparation of a 97/3 mixture of 4-methyl (C4-isomer) and 2-methyl substitued lauryldiamide (C2 isomer), also named hereafter C12 gelator 97/3

In a 1000 ml round bottom flask equipped with a mechanical stirrer and a condenser 127.13 g of methylglutarimide (0.7 mol) and 185.35 g of melted dodecylamine (0.7 mol, 1 éq.) was added. The suspension was heated to 130°C during 4 hours. The obtained product mixture contained an isomer4/isomer2 ratio of 52/48. This mixture was then cooled down to 100°C, then 400 mL of methylethylketone were added. The mixture was then heated to 80°C, then cooled down to room temperature, leading to a crystallization. The mixture was filtered and the solid was washed twice with 100 mL of ethyl acetate. The solid was suspended in 1.5 l of methylethylketone, the mixture was heated to 80°C, then cooled to 30°C and filtered. This solid was suspended in 450 ml of methylethylketone and heated to 80°C then cooled down to 30°C and filtered. The obtained solid (17.1 g, 8% yield) contained an isomer 4/ isomer 2 ratio of 97/3.

### Example 3a (comparative); Preparation of a 53/47 mixture of 4-methyl (C4-isomer) and 2-methyl substitued oleyldiamide (C2 isomer), also named hereafter C18 gelator 53/47

In a 1000 ml round bottom flask equipped with a mechanical stirrer and a condenser 63.57 g (0.5 mol 1 eq) of methylglutarimide and 185.35 g of Oleylamine (0.475 mol, 0.95 eq.) was added. The suspension was heated to 130°C during 4 hours. This mixture wass then cooled down to 80°C, then 350 mL of methylethylketone were added. The mixture was then heated to 80°C, then cooled down to room temperature, leading to a crystallization. The mixture was filtered and the solid was washed twice with 100 mL of ethyl acetate. The solid was suspended in 1.0 L of methylethylketone, the mixture is heated to 80°C, then cooled to 30°C and filtered. The filtrate was concentrated under reduced pressure to reach a volume of 400 mL, then heated to 80°C, then cooled down to room temperature, leading to a crystallization. The mixture was filtered, and the obtained solid contained an isomer 4/isomer 2 ratio of 70/30. The solid was suspended in 100 mL of methylethylketone, the mixture heated to 80°C then cooled down to 30°C and filtered. The obtained solid had an isomer 4/isomer 2 ratio of 87/13. The **filtrate** was let crystallize at room temperature, then filtered. The obtained solid had an isomer 4/isomer 2 ratio of 53/47.

### Example 3b (comparative): Reparation of a 65/35 mixture of 4-methyl (C4-isomer) and 2-methyl substitued oleyldiamide (C2 isomer), also named hereafter C18 gelator 65/35

In a 500 ml glass reactor equipped with a cooling system, 60.0 g (0.472 Mol 1eq) of methyl Glutarimide (MGI) and 126.0 g (0.472 mol 1eq) of oleylamine then 1.2 g of tBuONa was added. The reactor was then heated till 150°C and maintained at the reaction temperature for 3h. Then the reaction mixture was cooled down.

The solid was added into 400 ml of methylethylketone (MEK) and heated to 100°C. The solution was then cooled down and the obtained solid was collected by filtration and washed by ethyl acetate (2*300ml). The obtained solid was dried in vacuo to give 145.0 g of white solid, the yield was 78%, the purity was >98%.

NMR analysis showed that the product was obtained as a mixture of 2 isomers:
- N1-hexyl-4-methylpentanediamide (65 wt%),
- N1-hexyl-2-methylpentanediamide (35 wt%).

### Example 3c (according to the invention): Reparation of a 96/47mixture of 4-methyl (C4-isomer) and 2-methyl substitued oleyldiamide (C2 isomer), also named hereafter C18 gelator 96/4

In a 1000 ml round bottom flask equipped with a mechanical stirrer and a condenser 63.57 g (0.5 mol 1 eq) of methylglutarimide and 185.35 g of oleylamine (0.475 mol, 0.95 eq.) was added. The suspension was heated to 130°C during 4 hours. This mixture was then cooled down to 80°C, then 350 mL of methylethylketone were added. The mixture was then heated to 80°C, then cooled down to room temperature, leading to a crystallization. The mixture was filtered and the solid was washed twice with 100 mL of ethyl acetate. The solid was suspended in 1.0 L of methylethylketone, the mixture was heated to 80°C, then cooled to 30°C and filtered. This solid was suspended in 450 ml of methylethylketone and heated to 80°C then cooled down to30°C and filtered. The obtained solid (12.0 g, 6% yield) contained an isomer 4/ isomer 2 ratio of 96/4.

### Examples 4a to 4c: Preparation of gel compositions

All compositions were prepared in 20ml scintillation glass vials. Appropriate weights of the gelator powder and the oils or solvents were weighted on a microbalance. Total weights of gelators plus liquid oil or solvent were from 8 to 18g.

For examples 4a through 4c : Good dispersions and solubilizations of the gelators at high temperatures (90°C to 110°C) were obtained using a tip sonicator (Branson Sonifier® 450) 6.3mm diameter tip at a duty cycle set at 50%, power at 70%, with sonication durations set at 4 minutes. All samples were fluid, transparent and clear at end of the sonicating preparation.

Unless specifically mentioned otherwise all samples were left to cool back to room temperature on a bench. The qualitative visual verification of the gelation was done by simply upturning the vials. The gelation was considered successful if no flow was observed upon inverting the vial at room temperature.

The gelation process and gel strength of the samples were followed and quantified by the measurement of the gel's elastic modulus. Once sonicated the samples were introduced into the cup of a conical cylinder geometry in Ultra+ Kinexus® Rheometer from Malvern equipped with a temperature control unit. The samples were melted at 120°C under shear set at 100s⁻¹ immediately followed by a oscillatory collection of elastic modulus G' versus time (frequency of 1 Hz at a 0.1 % strain) as the temperature was quenched from 120°C to 25°C then remaining at 25°C using the Peltier temperature controlled stage.

### Example 4a: Gel composition comprising a vegetable oil (Rapeseed Oil) and either the gelator of example 1b (C6 gelator 65/35) or the gelator of example 1c (C6 gelator97/3)

The Rapeseed oil RADIA 6105 was purchased from Oleon and used for these tests. The C6 gelator (65/35) and C6 Gelator (97/3) concentrations tested ranged from 0.1 to 2wt%. Both C6 gelators displayed the ability to prepare gels in rapeseed oil. In particular, successful gelation in rapeseed oil was obtained with a quantity of gelator above 0.5 wt% for the C6 gelator 65/35 and above 0.2 wt% for the C6 gelator 97/3. The obtained gels resisted an upside-down flip and stick to the bottom of the vial without flowing. The gels were all perfectly homogenous.

The elastic modulus G' measured on the 2 wt% samples using a Kinexus Ultra+® Rheometer from Malvern using a conical cylinder geometry through the cooling step from 120°C to 25°C gave values of 436 000Pa for the C6 gelator 97/3 and 1000Pa for the C6 Gelator 65/35.

### Example 4b: Gel composition comprising a vegetable oil (Rapeseed Oil) and either the gelator of example 2a (C12 gelator 30/70) or the gelator of example 2b (C12 gelator 55/45) or the gelator of example 2d (C12 gelator 97/3)

The Rapeseed oil RADIA 6105 was purchased from Oleon and used for these tests. The C12 gelator (30/70), the C12 gelator (55/45) and C12 gelator (97/3) were tested at 2 wt% concentration. All three C12 gelators displayed the ability to gel this rapeseed oil. The obtained gels resisted an upside-down flip and stick to the bottom of the vial without flowing. The gels were all perfectly homogenous. The elastic modulus G' was measured on a Kinexus Ultra+® Rheometer from Malvern using a conical cylinder geometry through the cooling step from 120°C to 25°C and maintained at 25°C. The obtained gels in this rapeseed oil had G' values of 80 000Pa, 125 000Pa and 147 000Pa for respectively the C12 gelator (30/70), the C12 gelator (55/45) and the C12 gelator (97/3).

### Example 4c: Gel composition comprising a vegetable oil (Rapeseed Oil) and either the gelator ofexample 3a (C18' gelator 53/47) or the gelator of example 3b(C18' gelator 65/35) or the gelator of example 3c (C18'gelator 96/4)

The Rapeseed oil RADIA 6105 was purchased from Oleon and used for these tests. The C18'gelator (53/47), the C18' gelator (65/35) and C18' gelator (96/4) were tested at a 2 wt% concentration. All three C18' gelators displayed the ability to gel this rapeseed oil. The obtained gels resisted an upside-down flip and stick to the bottom of the vial without flowing. The gels were all perfectly homogenous. The gels elastic moduli G' were measured on a Kinexus Ultra+® Rheometer from Malvern using a conical cylinder geometry through the cooling step from 120°C to 25°C and maintained at 25°C. The obtained gels in this rapeseed oil had G' values of 17000Pa, 4000 Pa and 43500 Pa for respectively the C18' gelator (53/47), the C18' gelator (65/35) and the C18' gelator (96/4).

### Example 5: Use of C6 Gelator(65/35) (gelator of example 1b) in an Ethanol-based solution (S1) and of C6 Gelator(97/3) (gelator of example 1c) in an Ethanol-based solution (S2)

A solution (S1) of the C6 Gelator (65/35) at 25wt% in ethanol was prepared. Another solution (S2) of the C6 gelator (97/3) was prepared. A few droplets of these solutions (S1 or S2) (typically 0.025g of solution (S1 or S2) in 9.975g of the solvent or oil to be gelled up to 0.250g of solution (S1 or S2) in 9.750g of solvent or oil to be gelled) were introduced in the vials which were then capped and vortexed for 30 seconds. The samples were then left to rest on the bench. Successful gelation was recorded using this C6 Gelator solution (S1 ans S2) in rapeseed oil from 0.5 wt % effective concentration of C6 gelator (65/35) for solution S1 or C6 gelator (97/3) for solution S2. The use of the solutions (S1 and S2) eliminated the high temperature step and allowed gelation through simple mixing at room temperature (about 25°C). The use of solution S2 showed an improved gelling efficacy using C6 gelator (97/3) compared to the use of solution S1 (containing gelator C6 (65/35)) with an elastic modulus increase measured at 330 Pa (for S2) compared to 210Pa (for S1) at a 1 wt% effective concentration after resting for 30 minutes seconds post destructuration at 100s-1 shear rate for 15 minutes. All rapeseed oil organogels obtained with of C6 gelators were transparent and clear.

### Example 6: Gel composition comprising a methylated seed oil (methylated rapeseed oil) and either the gelator ofexample 3b (C18' gelator 65/35) or the gelator of example 3c (C18'gelator 96/4)

2 wt% gels were prepared in the methylated rapeseed oil RADIA 7956 from Oleon, with the C18' gelator (65/35) and the C18' gelator (96/4). Their respective elastic modulus G' were recorded at 1000Pa for the C18' gelator (65/35) and 3700 Pa for the C18' gelator (96/4). The obtained gels resisted to an upside-down flip, and were soft, slightly cloudy.

## Claims

1. A N-hydrocarbyldiamide composition comprising
a) a compound of formula (Ia) 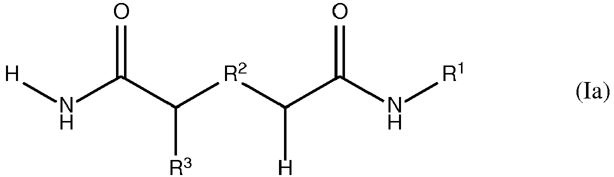 wherein
R¹ is selected from linear or branched or cyclic, saturated or unsaturated, hydrocarbon chains having from 1 to 40 carbon atoms,
R² is selected from linear or branched, saturated or unsaturated, hydrocarbon aliphatic chains having from 1 to 15 carbon atoms, and
R³ is selected from linear or branched alkyl groups having from 1 to 8 carbon atoms, and
b) a compound of formula (Ib) 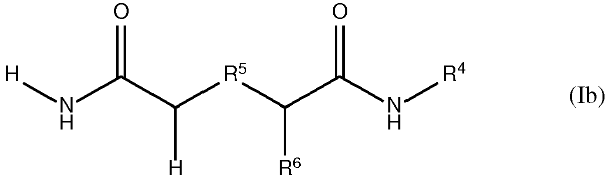 wherein
R⁴ is selected from linear or branched or cyclic, saturated or unsaturated, hydrocarbon chains having from 1 to 40 carbon atoms,
R⁵ is selected from linear or branched, saturated or unsaturated, hydrocarbon aliphatic chains having from 1 to 15 carbon atoms, and
R⁶ is selected from linear or branched alkyl groups having from 1 to 8 carbon atoms
said composition comprising the compounds of formula (Ia) and formula (Ib) in a molar ratio of at least 70:30.

2. The N-hydrocarbyldiamide composition according to claim 1 wherein the molar ratio of compound (Ia) to compound (Ib) is in the range of from 70:30 to 99:1.

3. The N-hydrocarbyldiamide composition in accordance with claim 1 or 2 wherein R¹ is equal to R⁴, R² is equal to R⁵ and R³ is equal to R⁶.

4. The N-hydrocarbyldiamide composition according to any of claims 1 to 3, wherein R² and/or R⁵, which may be the same or different, are linear alkylene chains, preferably C₁-C₄ alkylene chains, more preferably C₁-C₂ alkylene chains and even more preferably are methylene.

5. The N-hydrocarbyldiamide composition according to any of claims 1 to 4, wherein R¹ and/or R⁴, which may be the same or different, are selected from a linear, branched or unbranched, saturated or unsaturated, hydrocarbon group having from 2 to 40 carbon atoms, preferably from 4 to 32 carbon atoms, more preferably from 5 to 24 carbon atoms, even more preferably from 6 to 18 carbon atoms, said hydrocarbon group being preferably an aliphatic group.

6. The N-hydrocarbyldiamide composition according to one of claims 1 to 5 wherein the aggregated weight proportion of compounds (Ia) and (Ib) is at least 50 wt%, preferably at least 80 wt% of the total weight of the composition, and wherein even more preferably the compositions consists of compounds (Ia) and (Ib).

7. A process for manufacturing the N-hydrocarbyldiamide composition according to any one of claims 1 to 6, said process comprising
a) the reaction between an amine and a reactant selected from an imide, a diacid, a diester, a primary diamide, an ester amide, an acid ester, and thereafter
b) subjecting the reaction product obtained in step a) to at least two cycles of recrystallization and washing steps.

8. The process according to claim 7, comprising the reaction between at least one amine of formula (II) 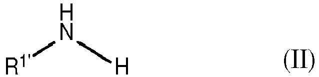 and at least one reactant selected from
- a cyclic imide of formula (III) 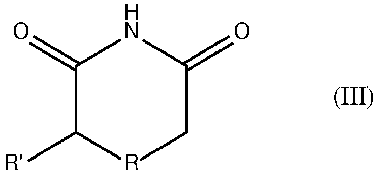
- a diacid of formula (IV) 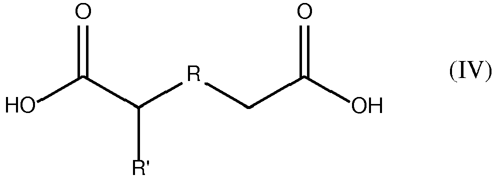
- a diester of formula (V) 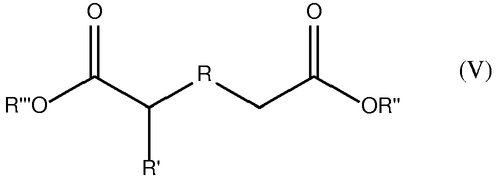
- a primary diamide of formula (VI) 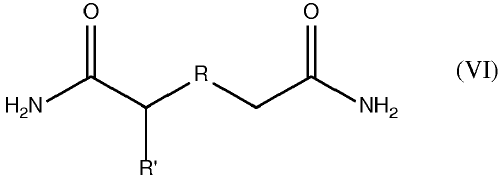
- an ester amide of formula (VII) 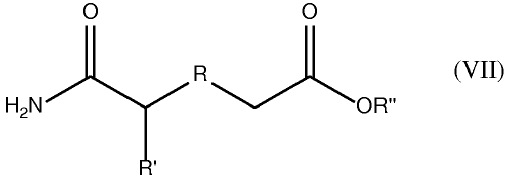 an acid ester of formula (VIII) 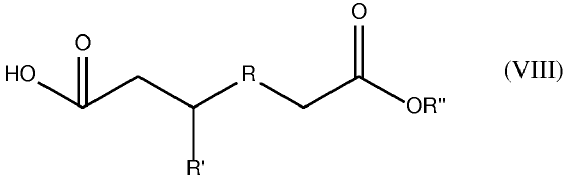
wherein
R is either R² or R⁵, R² and R⁵ having the same meaning as above in formula (Ia) respectively (Ib),
R' is either R³ or R⁶, R³ and R⁶ having the same meaning as above in formula (Ia) respectively (Ib),
R¹' is hydrogen or R¹ or R⁴, R¹ and R⁴ having the same meaning as above in formula (Ia) respectively (Ib),
and R" and R"', which may be the same or different, represent a hydrocarbon aliphatic chain, saturated or unsaturated, linear or branched, having from 1 to 40 carbon atoms.

9. A mixture (S) comprising the N-hydrocarbyldiamide composition according to any one of claims 1 to 6 and at least one solvent, said N-hydrocarbyldiamide composition being partially or fully solubilized in said solvent and said N-hydrocarbyldiamide composition being preferably fully solubilized in said solvent.

10. The mixture according to claim 9, wherein the solvent is a polar solvent selected from alcohols having from 1 to 6 carbon atoms, from acetal derivatives having from 2 to 12 carbon atoms, from alkyl esters having from 2 to 12 carbon atoms, and from mixtures thereof.

11. The mixture according to claim 9 or claim 10, wherein the N-hydrocarbyldiamide composition represent(s) from 1% to 75% by weight, preferably from 10% to 50% by weight, more preferably from 15% to 30% by weight of the total weight of the mixture.

12. Use of the N-hydrocarbyldiamide composition according to any one of claims 1 to 6 or of the mixture according to any one of claims 9 to 11, as a gelling agent.

13. A gel composition comprising a carrier and either the N-hydrocarbyldiamide composition according to any one of claims 1 to 6 or the mixture according to any one of claims 9 to 11, said gel composition being **characterized in that** the N-hydrocarbyldiamide composition according to any one of claims 1 to 3 is self-assembled in said carrier in order to provide jellification thereof.

14. The gel composition according to claim 13, wherein the carrier is selected from mineral oils, vegetable oils, animal oils, synthetic oils, linear or iso- alkanes, diesters having from 8 to 40 carbon atoms, glycerol, ethyl carbonate and dimethyl carbonate.

15. The gel composition according to claim 13 or claim 14, wherein the N-hydrocarbyldiamide composition in accordance with any of claims 1 to 5 represent(s) from 0.01% to 10% by weight, preferably from 0.05% to 5% by weight, more preferably from 0. 1% to 2.5% by weight, even more preferably from 0. 1% to 1% by weight, of the total weight of the gel composition, and/or wherein the carrier represents from 50% to 99.99% by weight, preferably from 60% to 99.95% by weight, more preferably from 70% to 99.9% by weight, even more preferably from 80% to 99.9% by weight, of the total weight of the gel composition.

16. The gel composition according to any one of claims 13 to 15, which comprises a surfactant.

17. The gel composition according to any one of claims 13 to 16, which is a battery electrolyte or a cosmetic product, such as a personal care product.

18. A formulation comprising the gel composition of claim 16 and additionally a biologically active ingredient.
